# EUROPEAN PATENT APPLICATION

(11) **EP 2 207 143 A2**
(43) Date of publication of application: **14.07.2010**
(21) Application number: 10150021.3
(22) Date of filing: 04.01.2010
(51) Int. Cl.: G07C 1/10, G07C 11/00

(54) **Method and apparatus for analysis and improvement of hand hygiene practices**

(30) Priority: 02.01.2009 US 319075
(71) Applicant: Gojo Industries, Inc., Akron, OH 44311 (US)
(72) Inventor: Dolan, Michael J., Akron, OH 44333 (US)
(74) Representative: Tetzner, Michael

(57) **Abstract**

A method and apparatus for analyzing the nature, location, position and orientation of hand hygiene dispensers within a facility and correlating that information with the usage experienced by each specific dispensers in order to correlate usage with physical characteristics of the dispenser, its location and position. Dispensers of various types are provided with data chips and acquire data correlating usage with the date and time of a dispensing cycle. The physical parameters associated with each dispenser are also known and recorded. All of this data is downloaded periodically into a database that assess such data in order to correlate usage of hand hygiene dispensers with parameters such as the nature of the dispenser, its location, position and orientation, time of day, work shift and the like. From this data, parameters can be defined that will optimize the use and positioning of hand hygiene dispensers within a facility such as a health care unit, hotel, restaurant or the like. According to one embodiment of the invention, the data is acquired by a reader or transponder that communicates with the data chip of the various dispensers using infrared or ultrasonic signal transmission. This acquired data is downloaded into a data processor for the analysis and optimization of dispenser positioning.

## Description

### TECHNICAL FIELD

The invention herein resides in the art of hand hygiene practices. More particularly, the invention relates to a methodology of improving hand hygiene practices. Specifically, the invention relates to a method of improving hand hygiene practices by removing and/or minimizing the physical obstacles and barriers to good hand hygiene practices. The invention particularly relates to a methodology of improving hand hygiene practices by assessing and quantifying physical impediments to good hand hygiene practices and removing such impediments in locations of interest.

### BACKGROUND ART

Good hand hygiene practices typically involve the washing, sanitizing, disinfecting or other means of cleaning one's hands prior to, during, or following a particularly activity. It is commonly known that disease is widely transmitted by hand contact with infected persons or objects. While good hand hygiene practices, such as the timely washing or sanitizing of one's hands, is important in everyday life to maintain health and vitality, it is acutely important in many industries such as the healthcare industry, food service industry, hospitality industry and the like. Recently, these industries have become increasingly aware of the need for good hand hygiene practices and, to a large extent, have addressed the issue with attempts to effect behavior modification of the persons involved in the industry. Educational programs, facility programs, along with periodic training and promotions have been a typical means for encouraging workers in these industries to adopt behavior conducive to and consistent with good hand hygiene practices. Such endeavors typically achieve at least temporary success. However, absent continual education reinforcement and periodic promotion of good hand hygiene practices, behavior modification is generally temporary and without lasting results.

It has been found that good hand hygiene practices are best achieved in physical environments that are conducive to those practices. In other words, where the opportunities for effecting hand cleaning/sanitation are made apparent and readily accessible, they are far more often used than when those opportunities are poorly located, spaced or timed. In other words, all else being equal, the likelihood for good hand hygiene practices to be engaged in an environment that is physically conducive and supportive of such practices is much greater than an environment in which little if any thought, apart from standard practices, has been given to the issue.

Indeed, it has been found in the past that the placement of hand sanitizing/cleaning apparatus in facilities of the various industries described above has been more the result of a continuation of common practice than one of analysis and assessment.

There is a need in the art for a method for analysis and improvement of hygiene practices that takes into account the impact of the environment upon those practices. There is indeed a need in the art for a methodology by which the placement of hand hygiene devices may be strategically effected in such a way as to optimize the use of such devices. Specifically, there is a need in the art for a methodology by which the placement of hand hygiene devices may eliminate the barriers to such practices that have previously been existent.

### DISCLOSURE OF THE INVENTION

In light of the foregoing, it is a first aspect of the invention to provide a method and apparatus for analysis and improvement of hand hygiene practices in which the location and positioning of hand hygiene dispensers is correlated with use.

Another aspect of the invention is the provision of a method and apparatus for analysis and improvement of hand hygiene practices wherein data corresponding to hand hygiene use is correlated with the position and location of hand hygiene dispensers.

Yet another aspect of the invention is the provision of a method and apparatus for analysis and improvement of hand hygiene practices wherein hand hygiene dispensers are located and positioned in an environment in manners that are conducive to and consistent with optimized usage of such dispensers.

Another aspect of the invention is the provision of a method and apparatus for analysis and improvement of hand hygiene practices in which the data is obtained innocuously.

Still a further aspect of the invention is the provision of a method and apparatus for analysis and improvement of hand hygiene practices in which the physical positioning of hand hygiene dispensers is determined by data correlating the usage of hand hygiene dispensers with their position and location.

The foregoing and other aspects of the invention that will become apparent as the detailed description proceeds are achieved by a system for improving hand hygiene practices, comprising; a plurality of dispensers of hand hygiene material at various locations, positions and orientations within a facility, each dispenser having a uniquely associated memory receiving and storing data corresponding to hand hygiene events; a transponder in selective communication with each said dispenser for reading said memory and retrieving said stored data from each of said plurality of dispensers; and a data processor in selective communication with said transponder for receiving said data from said transponder from each of said plurality of dispensers, and quantifying relationships between hand hygiene events and at least certain of said locations, positions and orientations and physical characteristics of said dispensers.

Other aspects of the invention that will become apparent herein are achieved by a method for improving hand hygiene practices, comprising: identifying dispensers of hand hygiene materials by location within a facility; acquiring usage data from said dispensers; correlating said usage data with locations within the facility; quantifying relationships between usage and location; and positioning and repositioning dispensers within the facility in accordance with said quantifications to optimize use of the dispensers.

Further aspects of the invention that will become apparent herein are achieved by a method for improving hand hygiene practices, comprising: establishing a first set of data comprising the nature and location of a plurality of dispensers of hand hygiene material; obtaining a second set of data from said plurality of dispensers of hand hygiene material regarding the dispensing of such material; correlating said first and second sets of data, thereby establishing a relationship between the nature and location of said dispensers and a tendency for their use; and employing said relationship in subsequent actions of locating or relocating said dispensers.

### DESCRIPTION OF DRAWINGS

For a complete understanding of the various aspects and techniques of the invention, reference should be made to the following detailed description and accompanying drawings wherein:
Fig. 1 is an illustrative side elevational view of a dispenser employed in association with the invention;
Fig. 2 is a plan view, in partial section, showing the placement of dispensers at various locations within a facility;
Fig. 3 is an illustrative view of various dispenser positions in association with a work station, such as a sink; and
Fig. 4 is a flow chart employing the method of the invention for improving hand hygiene practices.

### BEST MODE FOR CARRYING OUT THE INVENTION

In order to assess and improve usage of hand hygiene dispensers, it has been found that an assessment must be made of the use of such dispensers at their various physical locations. In the past, observational surveys have been used to measure hand hygiene compliance among health care workers, food service workers, and the like. However, observational surveys are time consuming and given to error, since the presence of an observer has the inherent effect of increasing usage by the workers. Alternatively, actual consumption of the dispensed product, be it a sanitizer, disinfectant, soap or the like, has also been employed for assessing the frequency of usage or hand hygiene episodes among such workers. Both types of data acquisition are insufficient for an assessment that seeks objective data in an unobtrusive manner, and which can tie the hand hygiene episodes to specific times and/or events.

The present invention contemplates the implementation of dispensers using, for example, an alcohol based hand sanitizer and including electronics uniquely associated with the dispenser, identifying the dispenser as to serial number and/or location, and receiving data from the dispenser corresponding to dispense cycles. Specifically, this data may include the details of the dispense event, including but not necessarily limited to, the date and specific time of day or work shift that each dispensing cycle or hand hygiene event occurred. Such data, for all dispensers in a facility, can then be gathered and transported to a processing unit such as a laptop computer, for assessment and correlation. This assessed correlated data may then be employed to position dispensers in a new facility, or reposition them in an old or existing facility to maximize their usage with respect to physical parameters.

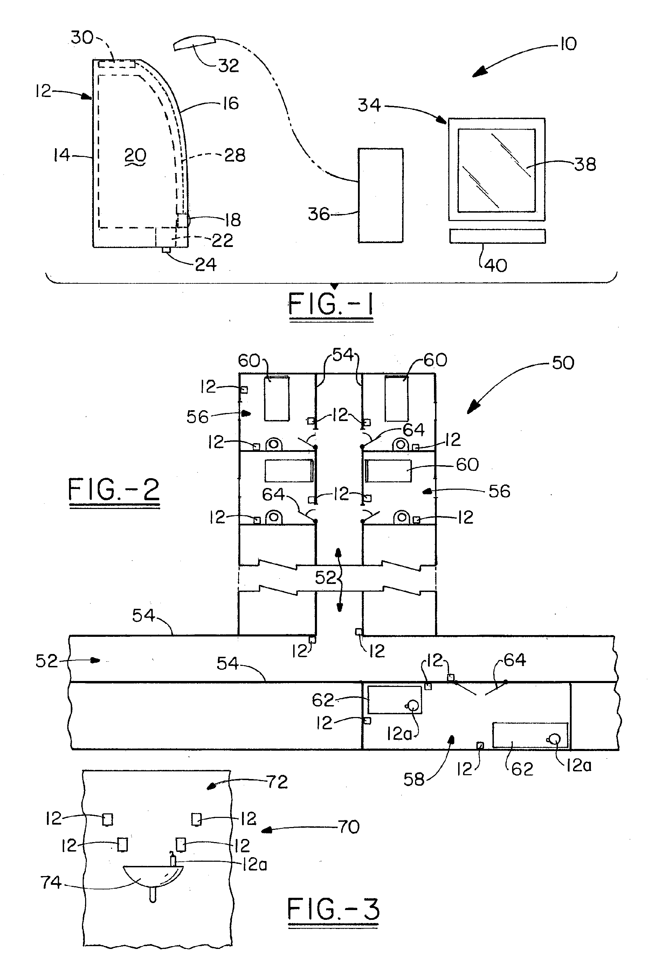

With reference now to the drawings and more particularly Fig. 1, an appreciation of the basic hardware employed with the concept of the invention can be obtained. As shown, a hand hygiene dispenser system used in accordance with the invention is designated generally by the numeral 10. The system 10 includes a plurality of dispensers 12, each including a housing 14, typically having a hinged front cover 16 to allow access to a substantially hollow interior thereof. An actuator 18 is provided in association with the dispenser 12, with the actuator 18 being of any of various natures and configurations. Those skilled in the art will appreciate that the actuator 18 may comprise a push or pull lever, push button, touch-free sensor, or the like. While the invention is generally described herein in the context of a system employing wall-mount dispensers, those skilled in the art will appreciate that it extends to a wide array of dispensers, including those mounted on a counter top, bed rail, or piece of equipment, by way of example, as well as free standing dispensers. The invention is not limited to any particular type of dispenser, whether permanent or semi permanent.

The interior cavity defined by the housing 14 is uniquely configured to receive and maintain a cartridge 20 that typically maintains therein an appropriate liquid soap, gel sanitizer, or the like. The cartridge 20 is typically of a disposable nature and will often include an integral pump 22 having a nozzle 24 extending therefrom, and from which the soap or sanitizer can be dispensed upon actuation of the actuator 18 and the implementation of control linkages and the like between the actuator 18 and pump 22. Those skilled in the art will appreciate that the pump 22 may be an integral portion of the disposable cartridge 20, or be part and parcel of the housing 14. Moreover, it will be readily appreciated that the invention extends to dispensers employing bulk reservoirs that may be refilled, as well as disposable and replaceable cartridges.

According to a preferred embodiment of the invention, an electrical conductor 28, such as a wire or printed circuit element is operatively interconnected between the actuator 18 and a data chip 30 maintained as part of the housing 14. While the invention is described with regard to a "hard wired" interconnection between the actuator 18 and data chip 30, it will be appreciated that radio frequency transmission or other means of communication might be employed. In any event, it is further contemplated that the data chip 30 will also include identification data, such as serial number, place of location, or the like with regard to the associated dispenser 12, such that any data received and maintained by the data chip 30 is correlated with a specific dispenser 12 of known nature, location, position and orientation. According to a preferred embodiment of the invention, the data chip 30 contains a clock, memory, and an infrared or ultrasonic transmitter/receiver in order to transmit data, be reset or cleared, and the like.

Also included as a portion of the hand hygiene dispenser system 10 is at least one reader or transponder 32, which is preferably of a hand held nature, separate from the plurality of dispensers, and adapted to communicate with the data chips 30 of the various dispensers 12 located throughout a facility. The reader 32 may be a wireless device that communicates with the various chips 30 by means of infrared, ultrasonic, non-contact electromagnetic or radio frequency signal transmission, although it is also contemplated that a plug-in type engagement might also be employed. It is further contemplated that the transponder 32 may be hard-wired, although a wireless system is preferred. In any event, hand hygiene control personnel or service technicians may employ the reader or transponder 32 to periodically access the data chips 30 of the various dispensers 12 and obtain therefrom the data stored therein, correlated with each specific dispenser. In that regard, the transponder 32 also includes an appropriate memory chip for storing the information from the data chips. Once the data from the chip 30 is obtained by the transponder 32, the chip 30 may be cleared or reset, for acquiring additional data that will subsequently be obtained by the reader 32.

Also included as a portion of the hand hygiene dispenser system 10 is a data processing system 34, which could be nothing more than a basic laptop or desktop computer system. It is also contemplated that the data processing unit may be a unique device customized to process the information from the transponder. As shown, the data processing system 34 would typically include a central processing unit 36, a cathode ray tube or video screen 38, and a keyboard 40, all interrelated in standard fashion. The central processing unit 36 is particularly adapted to communicate with the reader or transponder 32 to receive data from each of the various dispensers 12 located throughout a facility, and to assess, evaluate, and quantify that data in order to recognize patterns of hand hygiene practices in correlation with the nature and placement of hand hygiene dispensers 12 within a given facility.

Fundamental to the concept of the invention is the acquisition of data corresponding to hand hygiene events. For purposes of this invention, a hand hygiene event is determined by actuation of an actuator 18 of a dispenser 12. Such actuation is taken as corresponding to washing or sanitizing a user's hands which, in large part, is an extremely accurate measurement. Safeguards can be taken to ensure the integrity of the data. For example, if the actuator 18 is actuated multiple times within a set period of time, that event may correlate to a single hand hygiene event - - the user simply having acquired two volumes of soap or sanitizing gel to complete the event. It is also contemplated that the data chip may record the number of dispenses in each hand hygiene event as informative data.

With reference now to Fig. 2, a partial plan view of a facility can be seen as designated generally by the numeral 50. In accordance with the concept of the invention, the facility might be a hospital, hotel, restaurant, or any facility or area having a need for a plurality of hand hygiene dispensers. In any event, it is contemplated that the facility will be one where hand hygiene is deemed to be of significant importance. As shown, the facility is any defined area, but typically includes a plurality of corridors 52 defined by walls 54 extending therefrom. The walls 54 also serve to define rooms 56, such as patient rooms in a hospital or hospitality rooms in a hotel, and rooms 58, such as a kitchen, laboratory, operating room or the like. Within the rooms 56, and by way of illustration only, beds 60 or other objects are positioned in various arrangements and orientations. Similarly, in the rooms 58 other physical structures such as counters 62 are appropriately positioned. Of course, each of the various rooms 56, 58 is provided with one or more doors 64 allowing ingress and egress.

As shown in the illustration of Fig. 2, various hand hygiene dispensers, including wall mounted dispensers 12 or counter mounted dispensers 12a are positioned throughout the facility 50. The dispensers 12 may be free standing, mounted along the corridor walls 54, upon the walls 54 of the rooms 56, 58, and the dispensers 12a may be positioned upon the counters 62. In any and all such events, each of the dispensers 12, 12a is uniquely identified by serial number or the like and maintained within the data chip 30, such that the specific location of the dispenser is a part of the data retrieved by the transponder 32 and provided to the central processing unit 36. By way of illustration in Fig. 2, the dispensers 12 may be on the inside or outside of a door, to the right or left of a bed, in a corner, at the end of a room, near the corner of a corridor intersection, adjacent to or spaced from a counter 62, at various heights, along various travel paths related to infection control activities, and the like. In similar fashion, the counter top dispenser 12a may be of various positions on the counters 62, such as at a front edge, at a corner, at a back edge, and the like. A part of the data provided to the central processing unit 30 may also be traffic patterns within the system 50 that correlate with the placement of the dispensers 12 within the corridors 52.

With reference to Fig. 3, a further illustration of the placement of dispensers 12, 12a can also be obtained. Here, a sink arrangement is designated by the numeral 70, showing a sink 74 mounted upon an appropriate wall 72. Hand hygiene events are most typically, although not necessarily, expected in association with a sink. The dispensers 12 may be located at any of the various locations illustratively shown in Fig. 3, as wall mounted units, or a counter mounted unit 12a on the top of the sink 74.

Figures 2 and 3 have been presented for illustrative purposes only, to demonstrate that the placement of a dispenser 12, 12a may be at any of a wide variety of places within a facility, and are not meant to limit the possible dispenser locations contemplated by this invention.

Typically, the dispensers have been placed by tradition, rather than employing any analytical approach. While it may be commonsense to place a soap dispenser 12 or 12a in association with a sink 74, it is not known whether the dispenser is more likely to be used if it is placed at a certain height above the sink upon a wall, to the left or right of the sink, or upon the sink itself. Similarly, while it is known to place a hand sanitizer dispenser within or in association with patient rooms within a healthcare facility, it is not generally known whether the dispenser is more likely to be used if it is to the right or left of the bed, at what height on the wall, on the interior or exterior of a doorway, and the like. Similarly, as to dispensers 12 within traffic patterns in a facility, no conclusive data is known to exist or correlate traffic patterns with use, or traffic patterns with use in association with dispenser placement. The invention described herein allows for analytical investigation and analysis to determine optimal dispenser placement to promote hand hygiene compliance.

The invention contemplates not only an assessment of dispenser usage as a function of its placement, but also assessment of that usage as a function of the structure, operational features, and graphics of the dispenser itself. Knowing the specific nature of each dispenser from which data is acquired, along with its location, position and orientation, correlations of such parameters with usage can be readily made. Specifically, and by way of example, the invention addresses when and where manually actuated dispensers are more conducive to use than automatic touch-free dispensers, whether gel or foam is preferred, whether a large or small dispenser is preferred and what effects, if any, do dispenser graphics play in dispenser use. It is further contemplated that an assessment may be made regarding the nature and extent of semi permanent dispensers such as those adapted for temporary placement and use, such as on a bed rail or a piece of equipment. Assessments can be made as to the enhancement of hand hygiene practices realized as a result of the utilization of such semi permanent dispensers. While the concept of the invention described thus far relates to the placement of dispensers of various characteristics within a facility, similar data may be obtained and found to be useful with regard to personal dispensers that are carried upon a user, such as in a pocket, on a belt holster, by a clip and lanyard, or the like. Data correlating usage with the nature and placement of the dispenser on a user's person may also be beneficial in ensuring good hand hygiene practices and is contemplated as a portion of the invention described herein.

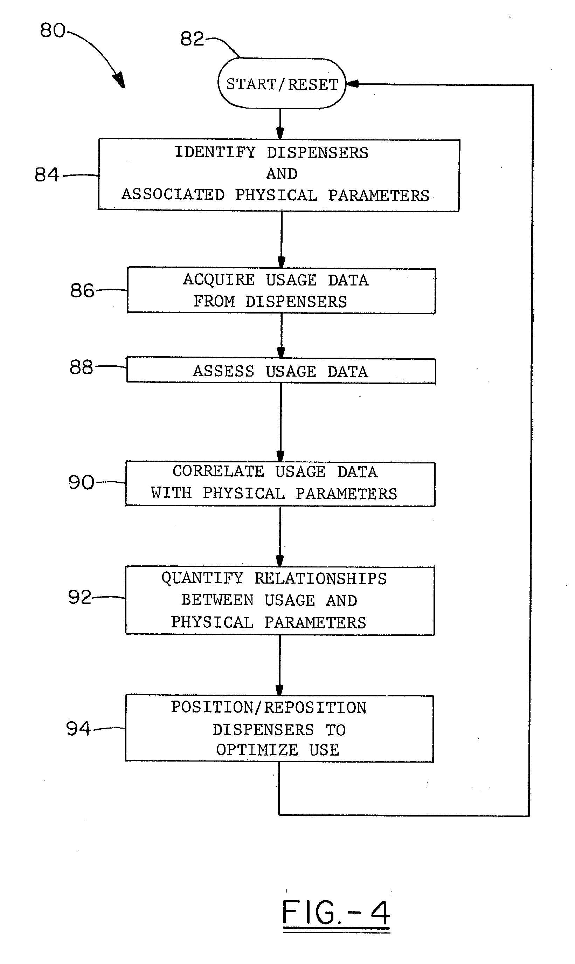

With reference now to Fig. 4, an appreciation can be obtained of the process of the invention which is shown in flow chart form and designated generally by the numeral 80. The process commences with a start/reset function 82 and passes to an activity block 84 where the various dispensers 12, 12a and their associated physical parameters are identified. In other words, each dispenser is identified by serial number or other identification, and is further identified as to its nature, location, orientation and position within a facility or upon a user. With each of the various dispensers 12, 12a having hand hygiene event recording mechanisms associated therewith, each such dispenser 12, 12a has within the associated data chip 30 a plurality of data correlating usage with time of usage for each hand hygiene event. That data is acquired at 86 by means of the transponder 32 such that, for any given test period, each dispenser 12, 12a within a facility, or otherwise in use, has data that is provided to the data processing system 34, where it is assessed as at 88. This data assessment correlates usage with time, date, work shift, and the like. Next, at 90, this usage data is correlated with the physical parameters associated with the dispenser 12, 12a. In other words the usage data is correlated with the location of the dispenser, its placement at that location as to height, counter top placement, wall mount placement, inside of a room, immediately outside of a room, to the left or right of a bed, sink, food preparation table and the like. The number of physical parameters associated with any dispenser 12 is substantially boundless, such that correlation of hand hygiene events with a multitude of physical parameters, and various combinations and permutations thereof, is possible.

At 92, a quantification is made of the relationships between usage and physical parameters. In other words, an assessment is made as to whether counter mount or wall mount units are more commonly used at a sink. Further assessment is made as to whether the units are more often used if placed at the right or left of the sink and, if a wall mount unit, the height at which the dispenser 12 is mounted above the sink. Similar quantifications are made with regard to placement within rooms, upon counters, within corridors, and further with regard to traffic patterns, work shifts and the like.

At 94, the quantification obtained is then employed to either position or reposition dispensers in an effort to optimize their use and, when such positioning or repositioning has occurred, further monitoring may take place and continual data may be obtained, assessed and quantified in order to continually improve upon the placement of dispensers in order to facilitate their use.

It should be appreciated that the concept of the invention is to place the dispensers of particular natures at those locations within a facility (or upon a person) which makes the use of such dispensers most likely. In other words, the placement of the dispensers makes them "user friendly," and removes the physical obstacles to use that might otherwise have been present. The concept of the invention is not to necessarily modify behavior of health care workers or the like, but to provide an environment in which good hand hygiene practices are easily engaged. The result will typically be an improvement in hand hygiene practices.

It should now be apparent that the concept of the invention includes the non-obtrusive acquisition of a volume of data related to dispenser usage and to subsequently overlaying that data onto a map of the associated facility, correlating dispenser usage with the nature, location, orientation and position of the dispensers within the facility. From this correlation, information regarding a relationship between nature, location, orientation and position of dispensers and their usage can be obtained and employed for repositioning dispensers in known facilities having similar parameters, or facilitating the placement of original dispensers in new facilities having similar characteristics. Several examples of the utilization and implementation of the concept of the invention are presented below.

### Example 1

In a first situation, all 126 wall-mounted soap and hand sanitizer dispensers in four wards in two acute-care hospitals were fitted with hand hygiene event recorders of the general type discussed above. The recorders were not visible to dispenser users and electronically determined, recorded and displayed a running total of the total number of hand hygiene events for each dispenser. The totalized number of each dispenser was manually read and transcribed to a datasheet at predetermined time intervals, typically 12 hours, which corresponded to the work shift times of the majority of healthcare workers in the wards. The number of hand hygiene events for a given time interval was calculated by subtracting the prior total from the current total of events recorded. This hand hygiene data was recorded over a period of 13 weeks.

At the same time, hand hygiene of the healthcare workers in the study wards was measured by a trained observer method. Four observers were uniformly trained to monitor and record hand hygiene opportunities and actual events, the ratio of the two providing a hand hygiene "compliance" value. Inter-rater reliability was determined to be greater than 95%. The standard observation methodology employed did not identify which dispenser was used by any individual healthcare worker.

The hand hygiene measurement data from the electronic monitoring devices in accordance with the invention was manually transferred to an engineering drawing of the four wards. This overlay of data to physical dispenser parameters allowed for identification and semi-quantitative assessment of several hand hygiene patterns. In addition, the data was statistically analyzed to further identify underlying patterns and relationships. For example, the ratio of soap to hand sanitizer events, effect of location, patient and staff loading, work shift and others were assessed. These analyses were then used to identify opportunities to improve hand hygiene rates. As a consequence of this analysis, several changes in the physical location, as well as the number of dispensers, were made to improve the level of hand hygiene in two wards.

In a similar manner, an attempt was made to analyze the hand hygiene measurement data obtained in parallel by the observation method. However, this data did not provide sufficient detail to identify opportunities to improve hand hygiene by physical changes. In particular, the lack of a unique identifier for each dispenser proved to be an insurmountable hurdle.

### Example 2

A study was conducted to determine the impact upon hand hygiene rates of placing wall-mounted hand sanitizer dispensers inside of patient rooms in addition to placement outside of the rooms. Two wards (one, an intensive-care unit and the other, a general medical-care unit) in an acute-care hospital were outfitted with the electronic hand hygiene recording devices described above. Recording was put in place for dispensers of hand sanitizer both inside (bedside) and immediately outside (corridor) of 23 patient rooms. Hand hygiene events were recorded for 60 days, during which 1,846 unique events were registered. The hand hygiene event data were statistically analyzed to determine patterns of use between inside and outside room placement of dispensers. To do this, the data was superimposed upon a physical map of the two wards of interest to correlate usage data with location data. It was found that 48% of the hand hygiene events occurred at the inside room dispensers, and a significant number of these events were additional hand hygiene, not simply a movement of practice from one area to another. Based upon these findings, additional dispensers were placed inside all patient rooms in the hospital.

### Example 3

In a 23-bed acute-care hospital ward, six patient rooms that were outfitted for contact isolation (contagious disease) precautions were equipped with a hand hygiene event monitoring system as presented above. Both soap and hand sanitizer dispensers were then monitored for seven months. The hand hygiene measurement data from the electronic monitoring devices was transferred to an overlay of the ward, as presented above. An analysis of the data showed that hand hygiene with soap and water was highly preferred over hand sanitizer use in these rooms due to the location of the hand soap and the overall flow of work needed to practice isolation precautions. It was further determined that physical placement of the hand sanitizer dispensers had relatively minor effect on usage rates, and, therefore, no changes in number or physical location of sanitizer dispensers were needed. Rather, resources were used to re-emphasize hand-washing practices in the contact precaution rooms. Here, where the norm was the use of soap for hand washing, rather than hand sanitizer, the data showed adherence to the norm despite placement of hand sanitizer dispensers. Accordingly, this undertaking demonstrated that in some situations, the placement of dispensers has little impact on their use. In such situations, there is no need for the expense and distraction of repositioning dispensers. Rather, attention may be given to other means for improving or maintaining good hand hygiene practices.

### Example 4

A two-phase study has been designed to determine the effect of physical location upon usage rates for soap and hand sanitizer in a food-service (chicken) restaurant. The restaurant will be divided into three zones: (1) food preparation, (2) counter service, and (3) drive-through service. A soap dispenser will be placed at the employees' sink in the food-preparation area, and hand sanitizer dispensers will be placed in the drive-through service (1) and front-counter service (2) areas. All dispensers will be equipped with the hand hygiene monitoring system as earlier described.

In the first study phase, a baseline of hand hygiene practices will be established by measuring the hand hygiene events when no hand sanitizer dispensers are present, *i*.*e*., only soap and water hand washing is practiced. This will represent typical current practice in a United States food service establishment. No hand washing will be recorded outside of the food preparation zone since there will be no sinks or dispensers in the other zones. Sixty days of baseline data will be recorded.

In the second study phase, hand sanitizer dispensers will be added to the other zones in the restaurant. An additional 60 days of data will then be collected for all three zones.

It is expected that analysis of the hand hygiene data will reveal that placement of hand sanitizer dispensers in the drive-through and front-counter service areas of the restaurant will result in a significant increase in hand hygiene events during the three busiest (most customers) one-hour periods of the day. Further, it is expected that no statistically significant change in the number of hand hygiene events will have occurred at the dispensers in the food-preparation sink area during these periods. If such proves to be the case, it is expected that the policy of the restaurant will be changed to include installation of hand sanitizer dispensers in multiple locations in order to increase the level of employee hand hygiene.

Other examples for the implementation of the invention will be readily apparent from the foregoing. Presently, it is contemplated that the method of the invention may be employed on cruise ships, with dispensers positioned along walkways, on observation decks, at various points in the dining facilities and the like. Indeed, the concept of the invention is adaptable to a wide variety of environments where good hand hygiene practices are important.

The invention has been described in the context of a plurality of dispensers positioned and monitored at the same time to acquire data that can be correlated with use. The invention also contemplates that a single dispenser of known characteristics, location, position and orientation may also be so monitored for data acquisition. That dispenser may then be repeatedly changed as to any of its parameters of interest and then monitored again for further data acquisition so that an array of data may be acquired, from which characteristics, features, locations, positions and orientations can be correlated with likelihood of use. Such information may then be employed positioning and/or repositioning of dispensers.

Thus it can be seen that the various aspects of the invention have been attained by the structure and method presented and described above. While in accordance with the patent statutes only the best known and preferred embodiments of the invention have been described in detail, the invention is not limited thereto or thereby. Accordingly, for an appreciation of the true scope and breadth of the invention reference should be made to the following claims.

## Claims

1. A system for improving hand hygiene practices, comprising;
a plurality of dispensers of hand hygiene material at various locations, positions and orientations within a facility, each dispenser having a uniquely associated memory receiving and storing data corresponding to hand hygiene events;
at least one transponder in selective communication with each said dispenser for reading said memory and retrieving said stored data from each of said plurality of dispensers; and
a data processor in selective communication with said transponder for receiving said data from said transponder from each of said plurality of dispensers, and quantifying relationships between hand hygiene events and at least certain of said locations, positions and orientations and physical characteristics of said dispensers.

2. The system for improving hand hygiene practices as recited in claim 1, wherein each said dispenser is selected from a group of permanent and semi permanent dispensers and has an actuator in operative communication with said memory.

3. The system for improving hand hygiene practices as recited in claim 2, wherein said transponder is a hand held portable unit.

4. The system for improving hand hygiene practices as recited in claim 3, wherein said transponder communicates with said dispensers through at least one of the following: infrared signals, non-contact electromagnetic signals, radio frequency signals, and ultrasonic signals.

5. The system for improving hand hygiene practices as recited in claim 2, wherein each said dispenser is uniquely identified as to at least certain of its location, position and orientation within the facility and said physical characteristics.

6. The system for improving hand hygiene practices as recited in claim 5, wherein said dispensers are taken from the group of wall mounted, counter top and personal use dispensers.

7. The system for improving hand hygiene practices as recited in claim 1, wherein said data processor identifies optimum parameters of said dispensers from among locations, positions, orientations and physical characteristics of said dispensers within the facility based upon said quantifying relationship between hand hygiene events and said locations, positions, orientations, and physical characteristics.

8. A method for improving hand hygiene practices, comprising:
identifying dispensers of hand hygiene materials by location within a facility;
acquiring usage data from said dispensers;
correlating said usage data with locations within the facility;
quantifying relationships between usage and location; and
positioning and repositioning dispensers within the facility in accordance with said quantifications to optimize use of the dispensers.

9. The method for improving hand hygiene practices as recited in claim 12, wherein said dispensers are further identified by a type of dispenser.

10. The method for improving hand hygiene practices as recited in claim 12, wherein said usage data includes time and date of usage.

11. The method for improving hand hygiene practices as recited in claim 14, wherein said location comprises parameters taken from at least the group of positional height, positional relationship to other objects, and positional relationship to travel paths, doors and wall corners.

12. The method for improving hand hygiene practices as recited in claim 15, wherein said travel paths are paths related to infection control activities.

13. The method for improving hand hygiene practices as recited in claim 15, wherein said usage data is acquired from said dispensers by signal transmission between said dispensers and a reader.

14. The method for improving hand hygiene practices as recited in claim 17, wherein said step of positioning and repositioning comprises identifying locations in the facility that have characteristics similar to those of locations that evidence high usage, and placing dispensers at such identified locations.

15. A method for improving hand hygiene practices, comprising:
establishing a first set of data comprising the nature and location of a plurality of dispensers of hand hygiene material;
obtaining a second set of data from said plurality of dispensers of hand hygiene material regarding the dispensing of such material;
correlating said first and second sets of data, thereby establishing a relationship between the nature and location of said dispensers and a tendency for their use; and
employing said relationship in subsequent actions of locating or relocating said dispensers.
